# FASCICULE DE BREVET EUROPEEN

(11) **EP 1 181 062 B1**
(45) Date de publication et mention de la délivrance du brevet: **12.05.2004**
(21) Numéro de dépôt: 00930937.8
(22) Date de dépôt: 26.05.2000
(51) Int. Cl.: A61L 2/14

(54) **SYSTEME ET PROCEDE DE STERILISATION PAR PLASMA A BASSE TEMPERATURE**
VERFAHREN UND VORRICHTUNG ZUR PLASMASTERILISATION BEI NIEDRIGER TEMPERATUR
LOW TEMPERATURE PLASMA STERILISING SYSTEM AND METHOD

(30) Priorité: 28.05.1999 CA 2273432
(43) Date de publication de la demande: 27.02.2002
(73) Titulaire: UNIVERSITE DE MONTREAL, Montréal, Québec H3T 1J4 (CA)
(72) Inventeur: MOISAN, Michel, Outremont, Québec H2V 2Z1 (CA); MOREAU, Stéphane, Montréal, Québec H2M 2M4 (CA); TABRIZIAN, Maryam, Longueuil, Québec J4J 5K3 (CA); PELLETIER, Jacques, 38400 Saint Martin D'Hères (FR); BARBEAU, Jean, Montréal, Québec H2T 2N9 (CA); YAHIA, L'Hocine, Pointe-Claire, Québec H9R 5T5 (CA)
(74) Mandataire: Boulinguiez, Didier
(86) Numéro de dépôt international: PCT/CA2000/000623
(87) Numéro de publication internationale: WO 2000/072889

(56) Documents cités:
- WO-A-92/15336
- WO-A-95/26121
- US-A- 4 643 876
- MOISAN M.; ZAKRZEWSKI Z.: 'Plasma sources based on the propagation of electromagnetic surface waves' J.PHYS.D. vol. 24, 1991, pages 1025 - 1048

## Description

### DOMAINE DE L'INVENTION

La présente invention concerne un système et un procédé de stérilisation d'objets, notamment des instruments et accessoires médicaux, en recourant au plasma gazeux, aussi appelé gaz ionisé. Les gaz utilisés pour former ce plasma n'ont pas besoin d'avoir une action stérilisante intrinsèque. Les propriétés stérilisantes selon l'invention résultent du passage du ou des gaz dans un champ électrique créant le plasma, champ fourni par exemple par des micro-ondes. L'avantage de l'invention réside dans la possibilité de traiter des objets thermosensibles ou thermolabiles à des températures inférieures à 50°C et en employant des gaz sans danger pour l'opérateur.

### ART ANTÉRIEUR

Les procédés de stérilisation dans le milieu hospitalier ont suscité beaucoup de controverse ces dernières années avec l'avènement d'instruments et accessoires en matériaux polymères généralement thermosensibles. En effet, l'autoclave est reconnu comme un appareil efficace pour la stérilisation, mais a le désavantage de nécessiter une température trop élevée (en l'occurrence supérieure à 121°C) pour permettre son application à l'égard de matériaux polymères. De plus, à la longue, l'utilisation de l'autoclave cause des dommages irréversibles aux objets en métal et modifie irrémédiablement certaines de leurs propriétés.

Afin de pallier ces limitations, des procédés de stérilisation par gaz ionisés, ou communément appelés plasmas gazeux, ont été développés. Ces procédés opèrent à des températures suffisamment basses pour permettre le traitement d'instruments et accessoires faits de matériaux polymères thermosensibles. Cependant, peu d'études ont été réalisées sur l'efficacité de ces procédés relativement aux mécanismes de destructions des micro-organismes.

L'utilisation de plasma comme agent de stérilisation a été proposée initialement vers la fin des années 60. Dans US 3,383,163, on décrit l'utilisation d'un champ électrique puisé pour générer un plasma d'argon ou d'azote, dans le but de stériliser les parois internes d'une fiole. Le procédé est tel que la stérilisation de la fiole a lieu directement dans la décharge.

La demande internationale WO 92/15336 décrit une méthode de stérilisation des objets, incluant l'activation d'un milieu gazeux substantiellement libre d'espèces chargées et l'exposition de l'objet à stériliser au mélange gazeux activé pour une période de temps suffisante pour obtenir la stérilisation complète de l'objet à stériliser.

L'intérêt d'une telle méthode s'est accru avec l'arrivée de stérilisateurs commerciaux tels que le Sterrad™, fabriqué et vendu par Johnson & Johnson, ou encore le Plazlyte™, fabriqué et vendu par Abtox. Il est toutefois connu que dans ces deux types d'appareils, le plasma n'a pas d'effet biocide, mais sert plutôt comme agent de détoxication en éliminant les résidus nocifs et en limitant l'effet oxydant du peroxyde d'hydrogène et de l'acide peracétique injectés sous forme de vapeur comme agent de stérilisation. On peut donc considérer que ces appareils sont basés sur la stérilisation par plasma, avec cependant la distinction que ladite stérilisation est réalisée grâce à l'addition d'espèces chimiques, et non pas par l'entremise des espèces activées du plasma lui-même.

Il serait donc avantageux de développer un système, ainsi qu'un procédé, de stérilisation totale utilisant des entités telles qu'un gaz peu coûteux et sans risque pour l'opérateur. Ce gaz ou mélange de gaz pourrait être activé à basse température par l'entremise d'un plasma, et appliqué sur un objet à stériliser, générant ainsi des espèces biocides permettant la stérilisation dudit objet, peu importe la nature du matériau dont est constitué cet objet.

### DANS LES DESSINS

La Figure 1 illustre un système de stérilisation selon la présente invention dans lequel le traitement a lieu dans la post-décharge;
La Figure 2 illustre un second système de stérilisation selon la présente invention.
La Figure 3 illustre le nombre de spores ayant survécu au traitement de stérilisation selon le procédé de l'invention en fonction du temps dans la post-décharge en utilisant de l'argon pur dans le tube à décharge.
La Figure 4 illustre le nombre de spores ayant survécu au traitement de stérilisation selon le procédé de l'invention en fonction du temps dans la post-décharge en utilisant dans le tube à décharge soit de l'argon pur, soit un mélange 5% O₂/ 95% Ar, l'introduction du gaz se faisant, dans les 2 cas, par le tube à décharge.
La Figure 5 illustre le nombre de spores ayant survécu au traitement de stérilisation selon le procédé de l'invention en fonction du temps dans la post-décharge en utilisant dans le tube à décharge soit un mélange de 15% O₂/ 85% N₂ (maximum de la concentration d'oxygène atomique), soit 2% O₂/ 98% N₂ (maximum du rayonnement UV de la molécule NO).
La Figure 6 illustre le nombre de spores ayant survécu au traitement de stérilisation selon le procédé de l'invention en fonction du temps dans la post-décharge, en utilisant dans le tube à décharge un mélange de 5% O₂/ argon.
La Figure 7 compare le nombre de spores ayant survécu au traitement de stérilisation selon le procédé de l'invention en fonction du temps dans la post-décharge en utilisant dans le tube à décharge un mélange de 5% O₂/ argon ou un mélange de 5% O₂/ azote.

### SOMMAIRE DE L'INVENTION

La présente invention a trait à un procédé de stérilisation d'objets comprenant l'exposition des objets à un plasma comprenant des espèces stérilisantes générées *in situ* par la soumission d'un flux gazeux comprenant entre 0.5 et 20% d'oxygène moléculaire à un champ électrique suffisamment intense pour générer le plasma, les espèces stérilisantes permettant la destruction de micro-organismes, ledit gaz n'ayant aucune propriété biocide avant son passage dans le champ électrique. Dans un mode de réalisation préférentiel, l'exposition des objets à stériliser a lieu hors de la zone d'excitation du plasma, i.e., dans la zone de post-décharge. Alternativement, l'exposition des produits au plasma a lieu dans une zone d'excitation du plasma.

Dans un autre aspect de l'invention, on retrouve un dispositif de stérilisation comprenant une source de plasma couplée à une chambre de stérilisation par un tube de décharge dans lequel est injecté un gaz ou un mélange de gaz générant éventuellement le plasma, la chambre contenant un objet à stériliser, et étant reliée à une pompe à vide pour entraîner les gaz dans la chambre et y maintenir une pression réduite. Préférablement, la source de plasma comprend un applicateur de champ électrique tel qu'un surfatron ou un surfaguide.

### DESCRIPTION DÉTAILLÉE DE L'INVENTION

La présente invention concerne un système et un procédé utilisant, en flux de gaz, un plasma, pour former des espèces atomiques et moléculaires ayant une action biocide. Ces espèces générées *in situ* permettent la stérilisation d'objets à des températures inférieures à 50°C, et ce, sans danger pour l'opérateur. Les gaz utilisés selon l'invention ne présentent pas de danger pour l'opérateur ou le patient, même dans l'éventualité où le temps d'aération après la fin de la stérilisation n'aurait pas été suffisant. En effet l'invention peut mettre en oeuvre des gaz qui conduisent à des espèces atomiques, radicalaires, moléculaires et des photons ayant un effet biocide seulement après avoir été soumis à l'action d'un champ électrique, résultant, par exemple, d'une tension continue ou radio-fréquentielle ou de micro-ondes, ces champs étant en régime permanent ou pulsé. Le procédé de l'invention peut donc être mis en oeuvre de façon isolée ou répétitive, par exemple dans une séquence de plusieurs étapes. Des exemples de telles étapes comprennent un gaz pulsé dans un champ électrique continu, un champ pulsé dans un gaz en flux continu, un gaz pulsé dans un champ pulsé de façon synchrone, un changement de gaz; ou des combinaisons de ces étapes.

La stérilisation est effectuée préférablement en post-décharge en flux gazeux dans divers gaz ou mélanges de gaz. Les gaz possibles comprennent l'azote, le néon, l'argon, le krypton, le xénon, l'hélium, l'oxygène, le monoxyde de carbone, le dioxyde de carbone, les NOₓ, se représentant un nombre entier sélectionné dans le groupe constitué par 1, 2 et 3, l'air et leurs mélanges, l'oxygène pouvant par ailleurs se retrouver sous forme moléculaire (e.g. O₂ ou O₃), ou comme élément d'un gaz (e.g. H₂O, CO, CO₂ etc.). A cause de leur faible nocivité, l'argon, l'azote et l'oxygène moléculaire O₂ représentent les gaz préférentiels. Des gaz plus nocifs comme les NOₓ ou le CO ne devraient idéalement être présents qu'en faible concentration afin de minimiser les risques pour la santé de l'opérateur et/ou des patients.

Il a par ailleurs été trouvé que la présence d'oxygène moléculaire (O₂) dans des proportions bien précises dans un mélange de gaz permet d'obtenir, en combinaison avec les rayons UV, une synergie telle que la période d'exposition de l'objet, en post-décharge ou non, est significativement diminuée. Plus spécifiquement, la concentration d'oxygène moléculaire doit se situer entre 0.5 et 20%. Le pourcentage d'oxygène dans une composition de flux gazeux donnée est ajusté en fonction de l'optimisation du rayonnement UV, rayonnement dont l'intensité agit directement sur le temps total requis pour une stérilisation complète. En d'autres mots, le pourcentage d'oxygène dans le flux gazeux est ajusté afin d'obtenir un rayonnement UV maximal, permettant de ce fait de réduire au minimum le temps de stérilisation. Cet ajustement peut être réalisé aisément par toute personne du métier versée dans la technique en utilisant la spectroscopie d'émission pour enregistrer l'émission UV ou toute autre méthode permettant d'obtenir des résultats similaires. Les résultats expérimentaux obtenus montrent que sous 0.5% et au-dessus de 20%, aucune synergie significative n'est alors observée. Préférablement, la proportion d'oxygène moléculaire dans le flux gazeux varie entre 2% et 5%. Un mélange 2% O₂/98% N₂ représente par ailleurs un mode de réalisation particulièrement avantageux.

Comparativement au traitement par autoclave, le présent procédé permet une stérilisation complète à des températures ne dépassant pas 50°C et sous une atmosphère réduite (de l'ordre de 1 mm Hg), au lieu des 121°C minimum et de la surpression d'une atmosphère requise dans un autoclave. Ceci signifie que la plupart des objets faits en partie ou en totalité de matériaux polymères, par exemple les pièces-à-main de dentiste, ou les cathéters, peuvent être stérilisés sans perdre leurs propriétés initiales par le procédé de notre invention.

Il est important ici de définir le terme "plasma" tel qu'entendu selon la présente invention. Un plasma comprend des ions et des électrons, libres de se mouvoir, qui sont produits en soumettant un gaz neutre à un champ électrique suffisamment intense pour accélérer les particules chargées, qui, en s'échangeant ensuite leur énergie par collisions, assurent ainsi l'entretien de l'état plasma. On établit parfois une distinction entre plasma et gaz ionisé par le fait que le plasma est généralement très chaud et ne contient que des espèces chargées, alors que le gaz ionisé, plus froid, comprend, outre les espèces chargées, des particules non chargées sous la forme d'atomes, de radicaux ou de molécules. Bien que la présente invention concerne essentiellement les gaz ionisés, le terme commun employé dans le champ d'activité de l'invention est "plasma", et c'est ce dernier qui sera utilisé dans la présente description.

Le domaine d'application visé par l'invention concerne la stérilisation d'objets de tous genres et fabriqués avec quelque matériau que ce soit. Généralement, les instruments et accessoires que l'on retrouve en milieu hospitalier sont faits de métal, de céramique et/ou de polymères. La présente invention permet plus particulièrement de remédier à certaines carences et limitations des systèmes actuels, à savoir:
- il est possible d'effectuer la stérilisation d'un objet sans l'exposer à des températures supérieures à 50°C, ce qui s'avère une caractéristique essentielle, par exemple, pour des objets manufacturés en tout ou en partie avec un matériau thermosensible tel qu'un matériau polymère;
- l'action stérilisante du procédé résulte d'atomes, de radicaux, de molécules et de photons générés *in situ,* soit produits initialement dans la décharge électrique, soit provenant d'interaction ultérieure dans la zone dite de post-décharge, mais dans les deux cas à partir de gaz qui, par eux-mêmes, ne possèdent pas d'activité ou d'action stérilisante. Les gaz ou mélanges de gaz choisis pour cet usage sont tels qu'ils sont sans danger pour l'opérateur en cas de mise en contact avec les effluents du traitement ou avec les objets à traiter, pendant ou immédiatement après la stérilisation.

La présente invention peut également être applicable à la stérilisation d'objets contaminés par des micro-organismes tels que des moisissures et champignons microscopiques, se trouvant par exemple dans des livres anciens.

Les particules, neutres ou chargées, à l'intérieur du plasma peuvent se trouver dans un état excité par rapport à leur état fondamental, donc dotés d'une énergie interne. Cette énergie interne peut être libérée spontanément en émettant un photon, ou encore par collisions avec d'autres particules ou avec une surface, par exemple celle des micro-organismes à inactiver. Les processus de collisions peuvent conduire à un échauffement de cette surface ou à des réactions chimiques avec elle, comme par exemple l'oxydation, menant à la formation d'un composé volatil liant le radical (atome ou molécule) incident sur la surface et un atome de cette surface. Les photons du domaine des ultraviolets (UV) sont particulièrement efficaces à ce titre puisque leur forte énergie leur permet de casser un grand nombre de liaisons chimiques. Les différentes espèces engendrées par une décharge électrique sont donc en mesure d'endommager le matériel cellulaire (e.g. protéines, ADN, enzymes etc.) et donc d'exercer un effet biocide.

Le procédé de la présente invention permet la destruction de micro-organismes tels que les bactéries, spores, virus, champignons et prions (agents transmissibles non-conventionnels) dans une courte période de temps, par l'action d'un champ électrique qui fait en sorte que le gaz auquel il est soumis engendre des atomes, des radicaux et des molécules dont l'action physico-chimique conjuguée, aussi bien en tant que particules que de photons émis par elles, entraîne la mort cellulaire de ces germes. Bien entendu, le procédé de l'invention n'interdit pas d'employer un gaz ayant intrinsèquement des propriétés stérilisantes, seul ou en combinaison avec des gaz dépourvus de propriétés stérilisantes.

La stérilisation par plasma selon la présente invention peut se faire soit à l'intérieur même de la décharge électrique par exposition directe au plasma, soit à l'extérieur de celle-ci. Dans ce dernier cas, on tire alors bénéfice des espèces transportées par le flux de gaz en provenance de la décharge dans un milieu appelé post-décharge. La post-décharge comprend peu d'espèces chargées comparativement à la décharge elle-même, mais contient à peu près autant d'espèces neutres actives que la décharge proprement dite. Le recours à la stérilisation dans le milieu de la post-décharge représente une mise en oeuvre particulièrement préférentielle pour 3 raisons:
- lorsque le plasma créé est de forte densité, par exemple avec des micro-ondes à 2,45 GHz, la température du gaz de la post-décharge est moins élevée que celle de la décharge elle-même, ce qui réduit substantiellement les risques de dommages aux objets stérilisés;
- comme il y a moins de particules chargées dans la post-décharge, les surfaces exposées sont moins ou pas du tout soumises à un bombardement ionique; et
- la post-décharge permet, pour un coût comparable, de disposer d'un volume de traitement plus grand qu'avec la décharge elle-même.

Lors d'une exposition en décharge directe, une température de traitement relativement basse peut être obtenue par l'utilisation d'une décharge en courant direct (DC), ce qui signifie un champ électrique d'intensité constante, ou l'utilisation de décharge radio-fréquentielles (RF), respectivement, dans des conditions de faible courant et de faible puissance RF, ou en maintenant une décharge pulsée.

Bien entendu, afin de réaliser une stérilisation efficace en post-décharge, l'opérateur devra préalablement s'assurer que suffisamment d'espèces actives, ou "stérilisantes" se retrouvent dans le milieu.

Des réactions peuvent aussi avoir lieu dans la post-décharge, qui créent de nouvelles espèces. Certaines de ces espèces neutres peuvent être dans un état excité et émettre des photons aléatoirement au cours de leur trajet dans la chambre de post-réaction.

Dans un autre mode de réalisation de l'invention, il est possible d'introduire un ou plusieurs gaz dans la décharge et, optionnellement ajouter dans la post-décharge même, un ou plusieurs gaz. Cette mise en oeuvre est intéressante lorsque l'on utilise des gaz comme le CF₄ ou le SF₆, dont la décomposition dans la décharge électrique entraînerait une usure importante du tube à décharge. Ces différentes possibilités permettent, le cas échéant, de maximiser l'action stérilisante du dispositif tout en minimisant les effets dommageables à long terme du traitement sur les objets à stériliser de façon répétitive.

Cette technique peut être utilisée de façon isolée ou répétitive dans des procédés séquentiels à plusieurs étapes, e.g., cycle de pompage suivi de l'alimentation en gaz plasma. Le choix du type de décharge alimentant la post-décharge dépend du type de radicaux recherchés. Ainsi, pour maximiser la concentration en azote atomique pour une puissance dissipée donnée dans la décharge, on aura recours à une décharge micro-ondes (≥ 300 MHz). Pour minimiser les coûts d'un tel dispositif, on peut alors choisir la fréquence des fours micro-ondes, soit 2,45 GHz. Alternativement, on peut utiliser l'exposition directe au plasma en autant que des fréquences du domaine des radiofréquences (de l'ordre de 100 MHz) soient utilisées puisque la densité du plasma est habituellement moins élevée dans ce cas.

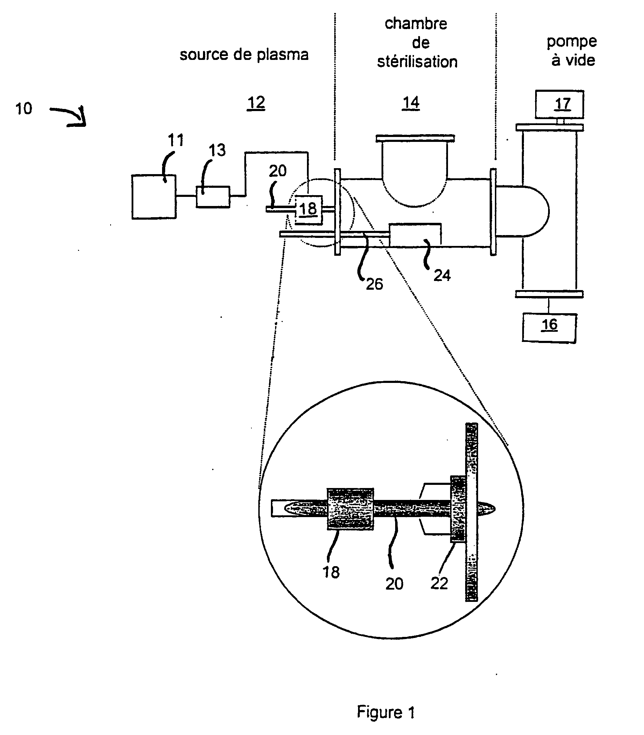

En se référant aux dessins qui illustrent des mises en oeuvre préférentielles de la présente invention, sans en limiter la portée, on retrouve à la Figure 1 un dispositif de stérilisation **10** comprenant une source de plasma **12**, une chambre de post-décharge ou stérilisation **14**, une pompe à vide **16** et une jauge de pression **17** qui permet de régler la pression du gaz. Le plasma est produit par une décharge micro-ondes à 2,45 GHz grâce à la propagation d'une onde de surface électromagnétique à une puissance maximale de 180W à partir d'un générateur **11**. La puissance est mesurée à partir d'un compteur **13**. Pour une puissance de l'ordre de 100 W ou plus, il est préférable, surtout si l'objet à stériliser est thermosensible, de refroidir le tube à décharge **20** par de l'air comprimé. L'onde de surface est excitée par un surfatron conventionnel **18**. Tout autre dispositif d'excitation de l'onde de surface, notamment ceux décrits dans US 4,043,940; US 4,810,933 ou dans *J. Phys. D.* : *Appl. Phys.* 1991, 24, 1025-1048 peut être utilisé, ce qui permet d'étendre le domaine possible des fréquences de quelques MHz à quelques GHz. Le diamètre du tube à décharge **20** par lequel le plasma est injecté dans la chambre **14** n'est pas critique, mais est préférablement choisi pour optimiser la production des espèces requises pour la stérilisation. Dans le mode de réalisation illustré à la Figure 1, le diamètre interne du tube est de 8 mm et sa longueur est de 300 mm. Toute personne du métier sera en mesure de déterminer aisément le diamètre approprié pour l'application envisagée. Le tube à décharge peut être fabriqué de n'importe quel matériau compatible avec l'opération du système, comme par exemple de la silice fondue.

Afin de prévenir la surchauffe des éléments scellants (joints toriques) disposés entre la chambre **14** et le tube **20**, surchauffe causée par les micro-ondes et le plasma, le diamètre du tube **20** est élargi à 30 mm à l'extrémité **22** adjacente à la chambre **14**. Le ou les gaz sont introduits dans le tube **20** à un débit ajusté à partir d'un débitmètre préalablement calibré (non illustré). Les gaz sont entraînés dans la chambre **14** grâce à la pompe **16** assurant un vide primaire (vide résiduel de 30 à 50 mTorr). Un étranglement plus ou moins important de la pompe permet de fixer la pression de gaz dans le réacteur. L'évacuation des gaz de la pompe **16** se fait sur l'extérieur de l'édifice, le cas échéant avec des filtres appropriés. La pression à l'intérieur de la chambre est préférablement abaissée à une valeur située entre 30 et 50 mTorr, et la pression du gaz entrant dans la chambre ajustée préférablement entre 1 et 7 Torr.

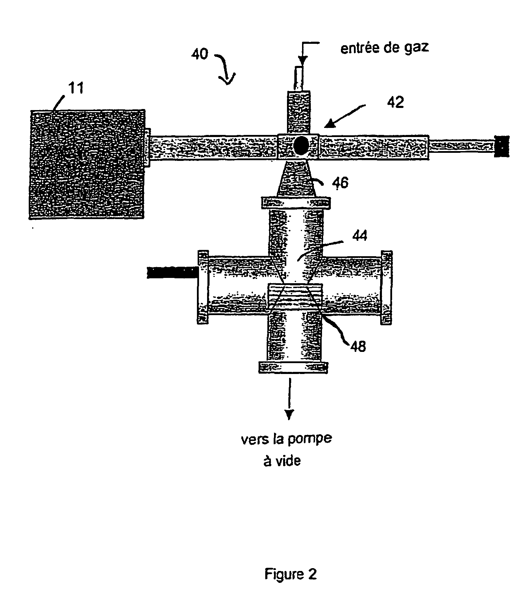

La chambre **14** possède un volume de 20 litres, et est fabriquée en Pyrex™, bien qu'elle puisse être constituée de tout autre matériau compatible avec le milieu. En fait, la chambre 14 peut être fabriquée entièrement ou partiellement en Pyrex™. Le Pyrex™, à cause de sa transparence, est particulièrement avantageux car il permet de pratiquer des observations par spectroscopie d'émission, de voir les effets du positionnement des objets à stériliser sur le flux de gaz, etc. De façon avantageuse, les objets à stériliser sont placés dans un support **24** préférablement fait en acier inoxydable. La forme du support est adaptée pour faciliter son nettoyage après usage, de même que la collection des objets stérilisés. Afin de contrôler la température dans le support **24**, on peut y faire circuler, en circuit fermé, un liquide de refroidissement à l'intérieur d'un conduit **26**, également en acier inoxydable. Dans un autre mode de réalisation de l'invention, représenté sur la figure 2, un second dispositif de stérilisation **40** a également été testé. Ce dernier comprend une source de plasma **42** comprenant un surfaguide, une chambre de post-décharge ou stérilisation **44** et une pompe à vide (non illustrée). Le plasma est à nouveau produit par une décharge micro-ondes à 2,45 GHz grâce à la propagation d'une onde de surface électromagnétique à une puissance maximale de 180 W transmise par le surfaguide. L'onde de surface est excitée par le surfaguide. Le tube à décharge 46 possède un diamètre interne de 40 mm. La chambre 44 est faite d'acier inoxydable dans laquelle est disposé le support 48. Les autres caractéristiques de ce dispositif sont relativement similaires à celles du dispositif illustré à la Figure 1. Il est toutefois intéressant de noter que dans ce mode de réalisation, le flux de gaz entre en contact perpendiculairement avec l'objet à stériliser, alors que dans le dispositif de la Figure 1, ce contact se fait parallèlement.

D'autres formes de sources de plasma ainsi que d'autres configurations et dimensions de chambre de stérilisation pourraient être avantageusement utilisées suivant le procédé de l'invention. Ainsi, la chambre de stérilisation pourrait être munie d'une porte d'accès pour y introduire et retirer les objets à stériliser. La même remarque vaut pour le support qui pourrait prendre des formes plus adaptées aux pièces à stériliser.

Le présent système a été testé en stérilisant des creusets contaminés avec des spores de référence utilisées conventionnellement pour le contrôle de l'efficacité des stérilisateurs commerciaux, en l'occurrence *Bacillus subtilis* var. *Niger* (ATCC 9372) et *Bacillus stearothermophilus* (ATCC 7953). La population initiale des spores est de quelques 10⁶ individus.

Selon le procédé de notre invention, une fois la chambre de post-décharge évacuée, un cycle complet de stérilisation dure environ 40 minutes et les objets à stériliser sont disponibles environ 5 minutes plus tard, soit le temps de remettre le réacteur à la pression atmosphérique. En comparaison, les procédés à l'oxyde d'éthylène nécessitent plusieurs heures de ventilation après le cycle de stérilisation, car il y a danger pour l'opérateur et le patient. Le procédé de la présente invention permet donc un gain de temps considérable par rapport à la méthode faisant appel à l'oxyde d'éthylène. De plus, les gaz utilisés pour le présent procédé sont sans effet toxique ou délétère sur les opérateurs et/ou les patients dans une pièce normalement aérée. Le temps de traitement selon la présente invention pourrait être réduit en augmentant la concentration d'espèces "stérilisantes" dans la chambre de stérilisation, par exemple en ajoutant une ou plusieurs sources de plasma, ou encore en optimisant la composition du mélange de gaz, le cas échéant, ou tout autre paramètre expérimental tel que la température, le débit du gaz, la pression à l'intérieur de la chambre etc.

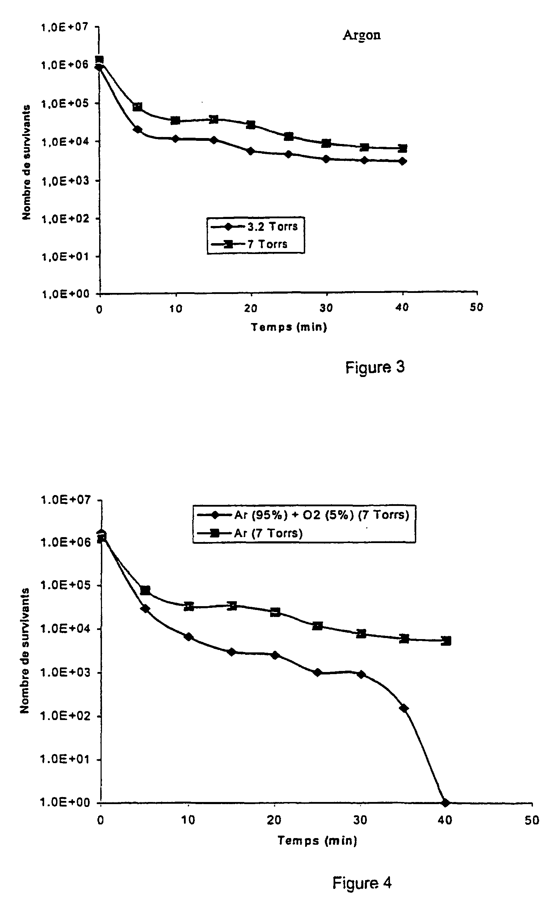

La Figure 3 montre les résultats obtenus, en post-décharge, avec le dispositif de la Figure 1 en utilisant simplement de l'argon pur, introduit dans le tube à décharge, à deux pressions différentes dans le réacteur, à un débit de 2 litres/minute aux conditions standard avec une puissance de 100 W. Comme on peut le constater, la stérilisation est loin d'être complète. Pour cette figure, chaque point sur le graphique représente la valeur moyenne obtenue pour 5 essais effectués dans des conditions similaires. Des résultats comparables ont été obtenus en remplaçant l'argon par de l'azote.

La Figure 4 montre les résultats obtenus, en post-décharge, avec le dispositif de la Figure 1 en utilisant soit de l'argon pur, soit un mélange 5% O₂/ 95% Ar. Dans les deux cas, l'introduction du ou des gaz se fait par le tube à décharge, avec un débit de 2 litres/minute aux conditions standard. La présence d'oxygène dans le mélange assure une stérilisation complète en 40 minutes. Pour cette figure ainsi que les suivantes, chaque point sur le graphique représente la valeur moyenne obtenue pour 6 essais effectués dans des conditions expérimentales similaires.

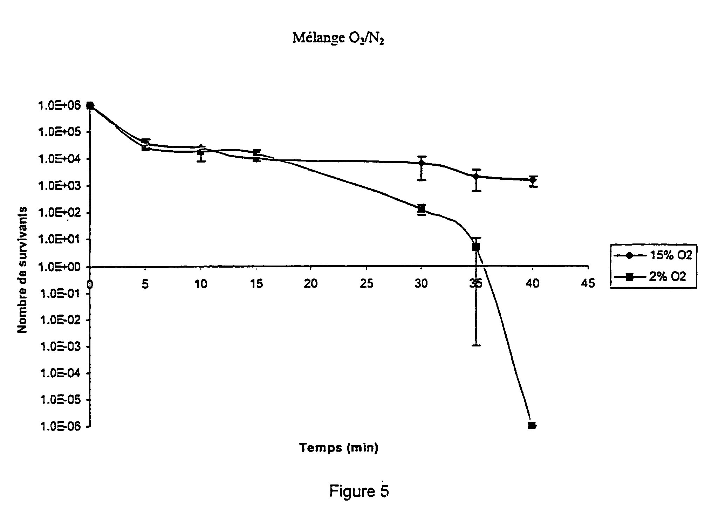

La Figure 5 illustre les résultats obtenus dans deux mélanges O₂/N₂. Dans le premier cas, la concentration d'oxygène a été optimisée afin de maximiser l'oxygène atomique (15% O₂/ 85% N₂), alors que dans le deuxième cas (2% O₂/ 98% N₂), la plus grande intensité possible de rayonnement UV provenant de la molécule NO (320-280 nm) a été recherchée. Pour déterminer le pourcentage d'O₂ conduisant à un maximum de concentration d'oxygène atomique, une méthode de titrage conventionnelle telle que décrite dans *Plasma Sources Science and Technology*, 1998, 7, 550-556 a été utilisée. Pour déterminer le maximum de O₂ conduisant à un maximum d'intensité UV, un enregistrement spectroscopique a été réalisé dans le domaine de longueurs d'onde autour de 320 nm. Dans les deux cas, la pression dans le réacteur était de 2,3 Torr et le débit de gaz de 500 mL/min aux conditions standard. Les résultats de la Figure 5 établissent clairement que la présence d'UV joue un rôle déterminant dans l'obtention d'une stérilisation complète et dans un temps relativement court, et qu'il existe un effet synergique entre l'oxygène et les rayons UV.

Les résultats illustrés aux Figures 4 et 5 montrent que l'obtention de la stérilisation complète varie selon le mélange de gaz utilisé, mais aussi de la proportion de chaque gaz dans le mélange lui-même.

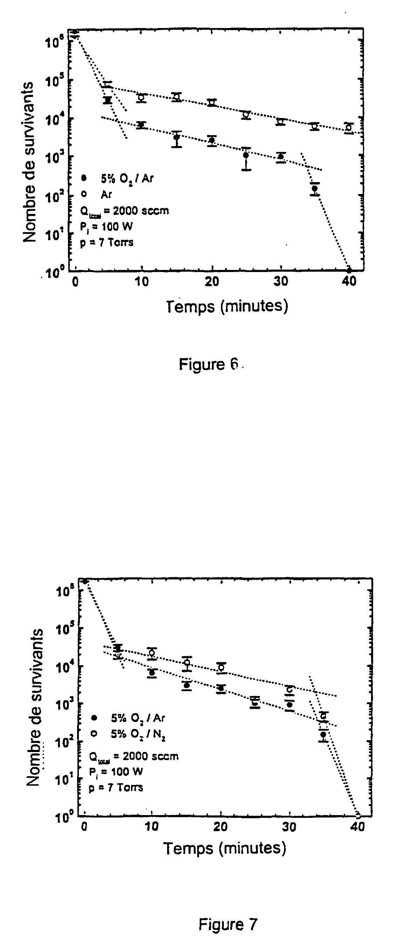

Il est bien connu que la présence d'oxygène dans la décharge augmente l'inactivation des spores par plasma. La Figure 6 illustre donc un mélange 5% O₂/argon, qui confirme le rôle de l'oxygène. Le point apparaissant à 10⁰ correspond à l'absence de détection de spores, i.e., on peut considérer que l'échantillon est stérile.

La Figure 7 compare le nombre de spores survivantes suite à l'utilisation de mélanges 5% O₂/95% Ar et 5% O₂/95% N₂ aux mêmes pressions et débits. Dans chaque cas, l'échantillon apparait stérile après environ 40 minutes. L'azote, étant donné son coût significativement inférieur à celui de l'argon, peut donc être privilégié.

Le débit de gaz dans la chambre à stérilisation peut varier selon la pression à l'intérieur de cette dernière, mais afin d'obtenir un rendement optimal de la stérilisation, il a été trouvé que les conditions préférentielles pour l'opération du procédé de l'invention requièrent un débit de gaz variant entre 0.5 et 2 litres par minute aux conditions standard, avec une pression de l'ordre de 1 à 10 Torr. De plus, le fait de porter les objets à stériliser à une certaine température fixe, par exemple à 50°C, par irradiation infrarouge ou autrement, semble activer la destruction des spores.

L'utilisation de plasmas à basses températures, typiquement inférieures à 50°C, sans l'addition d'entités chimiques très réactives, donc instables, représente donc une alternative sûre et efficace pour la stérilisation d'objets thermosensibles, tels que ceux fabriqués de matériaux polymères comme les endoscopes ou les cathéters.

Bien que la présente invention ait été décrite à l'aide de mises en oeuvre spécifiques, il est entendu que plusieurs variations et modifications peuvent se greffer aux dites mises en oeuvre, et la présente demande vise à couvrir de telles modifications, usages ou adaptations de la présente invention suivant, en général, les principes de l'invention et incluant toute variation de la présente description qui deviendra connue ou conventionnelle dans le champ d'activité dans lequel se retrouve la présente invention, et qui peut s'appliquer aux éléments essentiels mentionnés ci-haut, en accord avec la portée des revendications suivantes.

## Revendications

1. Procédé de stérilisation d'objets comprenant l'exposition des objets à un plasma dans une zone de post-décharge ou dans une zone d'excitation du plasma, ledit plasma comprenant des espèces stérilisantes générées *in situ* par la soumission d'un flux gazeux comprenant entre 0,5 et 20% d'oxygène moléculaire à un champ électrique suffisamment intense pour générer le plasma, les espèces stérilisantes permettant la destruction de micro-organismes, ledit flux gazeux n'ayant aucune propriété biocide avant son passage dans le champ électrique, ledit procédé étant **caractérisé en ce que** :
- le pourcentage d'oxygène moléculaire dans le flux gazeux est ajusté de façon à obtenir un rayonnement UV maximal.

2. Procédé selon la revendication 1, dans lequel l'exposition des objets audit plasma a lieu dans la zone de post-décharge.

3. Procédé selon la revendication 1 ou 2, dans lequel le champ électrique de la décharge est généré par une décharge micro-ondes.

4. Procédé selon l'une quelconque des revendications 1 à 3, dans lequel les espèces stérilisantes comprennent des photons, des radicaux, des atomes et/ou des molécules.

5. Procédé selon l'une quelconque des revendications 1 à 4, dans lequel le flux gazeux comprend, en plus de l'oxygène moléculaire, de l'azote, du néon, de l'argon, du krypton, du xénon, de l'hélium, de l'oxygène, du monoxyde de carbone, du dioxyde de carbone, des NOₓ, x représentant un nombre entier sélectionné dans le groupe constitué par 1, 2 et 3, de l'air, ou leurs mélanges.

6. Procédé selon la revendication 5, dans lequel le flux gazeux comprend, en plus de l'oxygène moléculaire, de l'azote, l'argon ou leurs mélanges.

7. Procédé selon l'une quelconque des revendications 1 à 6, dans lequel la proportion d'oxygène moléculaire dans le flux gazeux varie entre 2% et 5%.

8. Procédé selon l'une quelconque des revendications 1 à 7, dans lequel les micro-organismes comprennent les virus, spores, bactéries, champignons, moisissures ou prions.

9. Procédé selon l'une quelconque des revendications 1 à 8, réalisé à une température de 50°C au moins.

10. Procédé selon l'une quelconque des revendications 1 à 9, réalisé de façon isolée ou répétitive dans un procédé séquentiel à plusieurs étapes.

11. Procédé selon la revendication 10, dans lequel les étapes comprennent un gaz pulsé dans un champ électrique continu, un champ pulsé dans un gaz en flux continu, un gaz pulsé dans un champ pulsé de façon synchrone, un changement de gaz; ou un mélange de ces étapes.

12. Dispositif permettant la mise en oeuvre du procédé de stérilisation tel que défini dans l'une quelconque des revendications 1 à 11, comprenant une source de plasma couplée à une chambre de stérilisation par un tube à décharge dans lequel est injecté un gaz ou un mélange de gaz générant éventuellement le plasma, la chambre comprenant un objet à stériliser, et une pompe à vide pour entraîner les gaz dans la chambre et y maintenir une pression réduite, ledit dispositif étant **caractérisé en ce que** la source de plasma comprend un applicateur de champ électrique de type surfatron ou de type surfaguide.

13. Dispositif selon la revendication 12, dans lequel la chambre de stérilisation est fabriquée entièrement ou partiellement avec du Pyrex™.

14. Dispositif selon la revendication 12 ou 13, dans lequel la chambre de stérilisation comprend un support pour l'objet à stériliser, ledit support étant en acier inoxydable.

## Claims

1. Process for sterilization of objects, which comprises exposing said objects to a plasma in a post-discharge zone or in a zone of excitation of the plasma, said plasma comprising sterilizing species generated *in situ* by subjecting a gaseous flow comprising between 0.5 and 20% of molecular oxygen, to an electric field which is sufficiently intense to generate said plasma, said sterilizing species allowing destruction of microorganisms, said gaseous flow having no biocidal property prior to passage thereof through the electric field, said process being **characterized in that**:
- the percentage of molecular oxygen in the gaseous flow is adjusted in such a manner to give a maximal UV radiation.

2. Process according to claim 1, **characterized in that** the step of exposing said objects to the plasma is carried out in the post-discharge zone.

3. Process according to claim 1 or 2, **characterized in that** the electrical field of said discharge is generated by a microwave discharge.

4. Process according to claim 1, 2 or 3, **characterized in that** the sterilizing species comprise photons, free radicals, atoms and/or molecules.

5. Process according to claim 1, 2, 3 or 4, **characterized in that** the gaseous flow comprises, in addition to molecular oxygen, nitrogen, neon, argon, krypton, xenon, helium, oxygen, carbon monoxide, carbon dioxide, NOₓ with x representing an integer selected from the group consisting of 1, 2 and 3, air, or mixtures thereof.

6. Process according to claim 5, **characterized in that** the gaseous flow comprises, in addition to molecular oxygen, nitrogen, argon or mixtures thereof.

7. Process according to claim 1, 2, 3, 4, 5 or 6, **characterized in that** the proportion of molecular oxygen in the gaseous flow varies between 2% and 5%.

8. Process according to claim 1, 2, 3, 4, 5, 6 or 7, **characterized in that** the microorganisms include viruses, spores, bacteria, fungi, molds or prions.

9. Process according to claim 1, 2, 3, 4, 5, 6, 7 or 8, **characterized in that** the process is carried out at a temperature of 50°C or less.

10. Process according to claim 1, 2, 3, 4, 5, 6, 7, 8 or 9, **characterized in that** the process is carried out in an isolated manner or in a repetitive multi-step sequential process.

11. Process according to claim 10, **characterized in that** the steps include a gas pulsated in a continuous electric field, a field pulsated in a continuous gas flow, a gas pulsated in a field pulsated in a synchronous way, a change of gas, or a combination of these steps.

12. Device allowing the operation of the sterilization process according to any one of claims 1 to 11, comprising a plasma source coupled to a sterilization chamber by a discharging tube, **characterized in that** a gas or a gas mixture which eventually generates the plasma, is injected, the chamber containing an object to be sterilized, and a vacuum pump for carrying the gases into the chamber and for maintaining a reduced pressure, said device being **characterized in that** the plasma source comprises a surfatron or a surfaguide electric field applicator.

13. Device according to claim 12, **characterized in that** the sterilization chamber is constructed entirely or partially of Pyrex™.

14. Device according to claim 12 or 13, **characterized in that** the sterilization chamber includes a support for the object to be sterilized, said support being made of stainless steel.

## Patentansprüche

1. Verfahren zur Sterilisierung von Objekten, wobei man die Objekte einem Plasma in einer Nach-Entladungszone oder in einer Anregungszone des Plasma aussetzt, wobei das genannte Plasma sterilisierende Spezies umfaßt, die in situ erzeugt werden, indem ein Gasfluß, der 0,5 bis 20 % molekularen Sauerstoff umfaßt, einem elektrischen Feld ausgesetzt wird, das hinreichend intensiv ist, um das Plasma zu erzeugen, wobei die sterilisierenden Spezies die Zerstörung von Mikroorganismen ermöglichen, der genannte Gasfluß überhaupt keine biozide Eigenschaft vor seiner Einleitung in das elektrische Feld aufweist und wobei das genannte Verfahren **dadurch gekennzeichnet ist, daß**:
der Prozentsatz des molekularen Sauerstoffs im Gasfluß so eingestellt ist, daß eine maximale UV-Strahlung erhalten wird.

2. Verfahren gemäß Anspruch 1, worin die Objekte dem genannten Plasma in der Nach-Entladungszone ausgesetzt werden.

3. Verfahren gemäß Anspruch 1 oder 2, worin das elektrische Entladungsfeld durch eine Mikrowellen-Entladung erzeugt wird.

4. Verfahren gemäß einem der Ansprüche 1 bis 3, worin die sterilisierenden Spezies Photonen, Radikale, Atome und/oder Moleküle umfassen.

5. Verfahren gemäß einem der Ansprüche 1 bis 4, worin der Gasfluß, zusätzlich zum molekularen Sauerstoff, Stickstoff, Neon, Argon, Krypton, Xenon, Helium, Sauerstoff, Kohlenmonoxid, Kohlendioxid, NOₓ, wobei x eine ganze Zahl darstellt, ausgewählt aus der Gruppe bestehend aus 1, 2 und 3, Luft oder deren Mischungen umfaßt.

6. Verfahren gemäß Anspruch 5, worin der Gasfluß, zusätzlich zum molekularen Sauerstoff, Stickstoff, Argon oder deren Mischungen umfaßt.

7. Verfahren gemäß einem der Ansprüche 1 bis 6, worin der Mengenanteil des molekularen Sauerstoffs im Gasfluß 2 bis 5 % beträgt.

8. Verfahren gemäß einem der Ansprüche 1 bis 7, worin die Mikroorganismen Viren, Sporen, Bakterien, Champignons, Schimmel (Pilze) oder Prionen umfassen.

9. Verfahren gemäß einem der Ansprüche 1 bis 8, das bei einer Temperatur von mindestens 50°C durchgeführt wird.

10. Verfahren gemäß einem der Ansprüche 1 bis 9, das getrennt oder wiederholt in einem nacheinander in mehreren Stufen ablaufenden Verfahren durchgeführt wird.

11. Verfahren gemäß Anspruch 10, worin die Stufen ein in einem kontinuierlichen elektrischen Feld gepulstes Gas, ein in einem Gas in kontinuierlichem Fluß gepulstes Feld, ein in einem Synchron gepulsten Feld gepulstes Gas, ein wechselndes Gas oder eine Mischung dieser Stufen umfassen.

12. Vorrichtung zur Durchführung des in einem der Ansprüche 1 bis 11 definierten Sterilisierungsverfahrens, welche eine Plasma-Quelle, die an eine Sterilisierungskammer über eine Entladungsröhre gekoppelt ist, in welche ein Gas oder eine Gasmischung eingespritzt werden, die gegebenenfalls das Plasma erzeugen, wobei die Kammer ein zu sterilisierendes Objekt umfaßt, und eine Vakuumpumpe umfaßt, um das Gas in die Kammer einzuleiten und dort einen verringerten Druck zu halten, wobei die genannte Vorrichtung **dadurch gekennzeichnet ist, daß** die Plasma-Quelle einen Anwendungsgenerator für ein elektrisches Feld vom Surfatron- oder Surfaguide-Typ umfaßt.

13. Vorrichtung gemäß Anspruch 12, worin die Sterilisierungskammer gänzlich oder teilweise aus Pyrex™ hergestellt ist.

14. Vorrichtung gemäß Anspruch 12 oder 13, worin die Sterilisierungskammer eine Trägerunterlage für das zu sterilisierende Objekt umfaßt, wobei die genannte Trägerunterlage aus Edelstahl ist.
